# EUROPEAN PATENT SPECIFICATION

(11) **EP 3 130 563 B1**
(45) Date of publication and mention of the grant of the patent: **19.09.2018**
(21) Application number: 16160852.6
(22) Date of filing: 17.03.2016
(51) Int. Cl.: C02F 1/04, B01D 3/14, B01D 3/38, B01D 3/40, C02F 1/26, C02F 1/66, C07C 319/28, C07C 323/58, C02F 101/30, C02F 101/34, C02F 103/36

(54) **METHOD FOR TREATING METHIONINE PRODUCTION WASTE WATER**
VERFAHREN ZUR BEHANDLUNG VON METHIONINPRODUKTIONSABWASSER
PROCÉDÉ DE TRAITEMENT D'EAUX USÉES DE LA PRODUCTION DE LA MÉTHIONINE

(30) Priority: 10.08.2015 CN 201510487740
(43) Date of publication of application: 15.02.2017
(73) Proprietor: Bluestar (Beijing) Technology Center Co., Ltd., Beijing 100029 (CN)
(72) Inventor: NI, Huafang, 100029 Beijing (CN); ZHANG, Yi, 100029 Beijing (CN); PENG, Haitao, 100029 Beijing (CN); LI, Kaiyuan, 100029 Beijing (CN); HUANG, Ling, 100029 Beijing (CN); ZHANG, Chengjia, 100029 Beijing (CN); WANG, Hongwei, 100029 Beijing (CN); RANGEL OSALDE, Daniel, 100029 Beijing (CN)
(74) Representative: Manitz Finsterwald Patentanwälte PartmbB

(56) References cited:
- CN-U- 203 464 291
- SU-A1- 574 400

## Description

### FIELD OF THE INVENTION

The present invention relates to a chemical process system and method, in particular to a method and a system for treatingindustrial waste water.

### BACKGROUND OF THE INVENTION

Methionine is one of basic units that constitute protein, is widely applied and can be used as medicine,nutrient,food additive,feed additive, etc.

0.5 ton of waste water can be generated in order to produce one tone of methionine and contains organic acids such as acrylic acid, acetic acid and maleic acid, aldehyde such as acrolein and formaldehyde, and organic materials such as allyl alcohol and hydroquinone. The total content of the organic materials can reach about 5-20%.

This industrial waste water cannot be treated by using a microbiological method due to excessive high COD or organic material concentration. The existing method is to burn the waste water, i.e., spray the waste water into a combustion area through high pressure sprayer and then burn the organic materials in the waste water. Since the main component in the waste water is water, the water therein is gasified when the organic materials are thoroughly burned and a great amount of nature gas is consumed. In China which is in shortage of energy, a low energy consumption method is needed for treating methionine production waste water.

Methionine waste water treatment is disclosed in SU 574 400 A1 and CN 203 464 291 U.

### SUMMARY OF THE INVENTION

The present invention provides a new method for treating methionine production waste water, aiming at solving the existing problems of high energy consumption and low material utilization ratio

The method for treating methionine production waste water provided by the invention comprises the following steps: 1) using an extraction agent to extract the waste water, and performing separation to obtain a first organic phase and a first water phase; 2) rectifying the first organic phase to obtain acrylic acid from a side line of a stripping section, obtain heavy components from a bottom of a column, and obtain an extraction agent containing light components from the top of the column; 3) treating the extraction agent containing the light components with a light component removal column, and performing separation to obtain the light components and the extraction agent; 4) removing formaldehyde from the first water phase; and 5) performing biochemical treatment to the first water phase.

In one specific embodiment, the light components and/or heave components obtained by separation in Step 2) are burned. Another method is to esterify the heavy components and then to perform separation.

Preferably, before Step 4), steam stripping is performed to the first water phase to recover the extraction agent.

In one specific embodiment, in Step 4), formaldehyde is saccharified by adding alkali and heating.

In another specific embodiment, in the Step 4), formaldehyde is removed by using a formaldehyde saccharifying method and the saccharifying is performed in a steam stripping column.

In one specific embodiment, before step 3), firstly alkali solution is used to remove organic acid in the extraction agent containing light components.

By using the method provided by the present invention, 50-80% of organic materials can be recovered from methionine production waste water, the material utilization ratio is improved, a small amount of separated organic waste only needs to be burnt, the waste water containing a small amount of impurities can be treated through a microbiological method, and the entire method can reduce the energy consumption by more than 60%.

### DESCRIPTION OF THE DRAWINGS

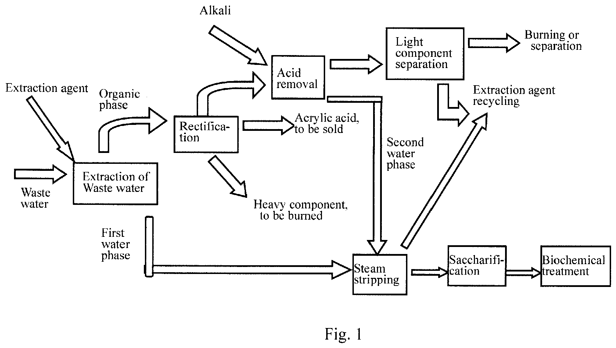

Fig. 1 is a flowchart of a waste water treatment method in an exemplary embodiment of the present invention.

### DESCRIPTION OF THE EMBODIMENTS

The operation of one specific embodiment of the present invention will be described below step by step. One skilled in the art can make changes to some technical details according to the common knowledge or common means without going beyond the technical concept of the present invention. Therefore, the protection range of the present invention shall be determined by the definition in claims and equivalent substitutions thereof.

### Extraction

This step is a basic step to separate waste water and organic materials therein. Through extraction, not only can most acrylic acid be recovered, but also most other organic materials are taken out from water, thereby relieving the workload of subsequent water treatment. Preferably, extraction agent for the present invention can be selected from a group of MIBK, DIBK, ethyl acetate, isopropyl acetate, isobutyl acetate, methyl acrylate, ethyl acrylate, butyl acrylate, dimethylbenzene, toluene, heptane, hexahydrotoluene, isobutyl ether. The most preferably, MIBK is selected and used because the residue of the extraction agent in a water phase is the fewest when this extraction is used.

Extraction is performed in an extraction column. Extraction conditions can be as follows: the weight ratio of extraction agent to waste water is 1:3-10:1; extraction temperature 10-60°C; stirring time 15-150 minutes; standing time 15-150 minutes; extraction times 1-5. Preferred conditions are as follows: the weight ratio of extraction to waste water is 1:2-3, temperature 10-30 °C, stirring time 15-20minutes, then standing time 15-30 minutes every time, and preferred repeated operation times are 3-5.

By adopting the above-mentioned preferred extraction conditions and MIBK, all formaldehyde remains in the water (first water phase) through extraction, the organic phase (first organic phase) has hardly any formaldehyde, and organic acid is distributed in both the water phase and organic phase.

### Rectification

The first organic phase is rectified by using a rectifying column. The first organic phase therein mainly contains extraction agent, and additionally contains acrylic acid, acetic acid, acrolein, allyl alcohol and maleic acid. Through rectification, acrylic acid is recovered from the side line of a stripping section. Maleic acid is recovered from the bottom of a column. Light components are recovered from the top of the column, which contain extraction agent, acrylic acid, allyl alcohol, acetic acid and the like. In case of using MIBK, a small amount of water in the first organic phase and extraction agent are azeotropic and are steamed out together.

The purity of acrylic acid separated in this step reaches more than 98% and acrylic acid can be directly used as an industrial raw material. Heavy components recovered from the bottom of the column mainly contain maleic acid, as well as other organic acids, such as a small amount of acrylic acid. Such acids are difficult to separate and can be sent for incineration, and the steam generated thereby can be used as heat source. As a more preferable method, such organic acids can be sold as industrial raw materials. Although the heavy components are in a mixture, the mixture is not complicated(usually are a few acid homologs) and rather stable and has certain industrial applicability. One separating method is reacting these acids with the onematerial to produce different products. Usually, those products are more easily separable than acid homologs. For a typical example, esterification is performed to enable organic acid mixture to react with a kind of alcohol under certain esterification conditions to produce ester which can be separated more easily and has industrial applicability.

In another preferred embodiment, MIBK is selected as extraction agent, and a proper amount of water is added into the column in step 2), such that MIBK and water form azeotrope, which is steamed out from the top of the column and does not contain acetic acid. This is the special advantage obtained by selecting MIBK as extraction agent. In this embodiment, a corresponding amount of water is introduced according to the amount of extraction agent, the temperature of the column is well controlled and most of extraction agent can be steamed out from the top of the column in the form of azeotrope. A small part of extraction agent carrying with acetic acid can be separated out from the upper side line of the column. Thereby, the first organic phase is separated into four parts in the rectifying step, including extraction agent-water azeotrope steamed out from the top of the column, extraction-acetic acid mixture separated from the upper side line of the column, an acrylic acid and heavycomponents separated from the middle side line and bottom of the column respectively. By adopting this embodiment, the workload of removing acetic acid by adding alkali can be greatly relieved, the distillate from the upper side line is single in components and only contains acetic acid and extraction, acetic acid can be removed by using a small volume of aqueous alkali, and the obtained aqueous sodium acetate can be concentrated to produce industrial sodium acetate and is not necessarily abandoned as waste water.

### Light component treatment

The purpose of this step is to recover extraction agent from light components coming from the top of the rectifying column, and this step is usually performed in a light component removing column. After light components with smaller boiling point or density than the extraction agent are separated out from the top of the column, the remaining is mainly extraction agent can be cyclically used. The light components removed in this step are burned and the produced steam is as a heat source.

Under the circumstances that the above-mentioned light components contain acetic acid, the boiling point of acetic acid is close to the boiling point of extraction agent, and thus the acetic acid is difficult to separate from the extraction agent. Preferably, it is removed from the extraction agent. Otherwise, acetic acid will be enriched in the extraction agent during extraction and equipment corrosion will be caused. One means is adding water before feeding in the light component removing column for rectification to introduce acetic acid into the water phase, the separated water phase(a second water phase) can be combined with the first water phase and then subsequent treatment is performed. Another preferred means is adding an aqueous alkali, preferably sodium hydroxide, into the light components to neutralize the acid therein, converting the same into sodium salt, with the latter entering into the water phase. Since the water phase does not contain organic acid, the amount of the extraction dissolved in water is smaller.

### Water phase treatment

The water phase (the first water phase) coming from the extraction step contains all formaldehyde, part of organic acids and a small amount of extraction agent. The second water phase coming from the light component treatment step also carries basically the same amount of extraction agent. Generally the waste water coming from the two sources are merged and then treated.

With respect to the treatment of the water phase, one specific embodiment of the present invention is to treat the organic materials in the water phase by aeration. Since what exist in the waste water are small-molecular organic compounds, these organic materials can be completely oxidized by aeration for a long period. However, this method has the disadvantage of long operation time, because the speed of thoroughly oxidizing organic acid such as acetic acid and acrylic acid into carbon dioxide and water is slow.

A micro biological degradation method is preferred, which has the advantages of low energy consumption and fast speed. Under the situation that formaldehyde is removed, degradation can be completed within 20 hours.

When the micro-biological degradation method is used, preferably formaldehyde in water is removed in advance because a certain amount of formaldehyde will kill part or all of microorganisms and thereby the efficiency of microorganisms is slowed down or lost. In one embodiment, formaldehyde is removed by using a sulfonation method, which is to add sodium hydrogen sulfite into the waste water to convert formaldehyde into rongalite. This method has the advantages of high formaldehyde removal rate, no heating and good product biodegradability, but has the disadvantage of high cost and is only suitable for low concentration formaldehyde. In another embodiment, formaldehyde is removed by using a urotropination method, which is to feed ammonia water into waste water to convert formaldehyde into hexamethylenetetramine (commonly known as urotropin). This method has the advantages of good formaldehyde removing effect (above 99%), exothermic reaction, fast reaction speed and low cost, but has the disadvantage that the difficulty in controllingammonia nitrogen concentration during subsequent treatment is increased due to the introduction of ammonia nitrogen.

As a preferred method of the present invention, formaldehyde is removed by using saccharifying method. In this method, a basic catalyst is fed into waste water to convert formaldehyde into hexanose. Alkali is not only used as catalyst, but also as a reactant. Hydroxyl is consumed in saccharifying reaction. Usable alkali includes calcium hydroxide, potassium hydroxide and sodium hydroxide. Preferably, sodium hydroxide and calcium hydroxide are selected as mixed saccharifying agent. The pH value is controlled within 10-12 by using sodium hydroxide, and then a consumption amount of calcium hydroxide is added. The mass ratio of formaldehyde to calcium hydroxide is 10:1 to 1:10, the most preferably, 5:1 to 1:2. Saccharifying temperature is controlled to be within 60-120°C, the most preferably, 70 to 100 °C, saccharifying time can be 20-170minutes, preferably 20-70minutes. By adopting the most preferred saccharifying conditions, 99% of formaldehyde can be removed. This method has the advantages of low cost and fast reaction speed.

In one more preferred embodiment, before water containing formaldehyde is removed according to the above-mentioned method, the waste water is subjected to a steam stripping process once again to recover extraction agent therein.

In one more preferred embodiment, steam stripping and formaldehyde saccharifying are simultaneously performed. Specifically, a required amount of alkali is firstly dissolved in waste water in the column in advance, the waste water is vaporized in the steam stripping process, and steam carrying with organic materials goes upwards to the top of the column. Saccharification is performed in the column and aldehyde in waste water is consumed.

Fig. 1 illustrates a flowchart of a waste water treatment method in an exemplary embodiment of the present invention.. In this embodiment, after waste water coming from a methionine production device is extracted, the organic phase is rectified by the rectifying column to form three parts, including heavy components from the bottom of the bottom of the column,which are to burned to generate by-product steam; acrylic acid from the side line, which can be sold as industrial materials; distillate from the top of column,which is mainly extraction agent, is added with alkali for acid removal and then is subjected to the treatment by the light component removing column to remove light components, wherein the remaining extraction agent is cyclicallyutilized. The extracted water phase is firstly subjected to steam stripping to obtain extraction agent, then alkali is added to saccharify allof formaldehyde, and waste water is finally fed to perform biochemical treatment.

The present invention abandons the existing means of burning methionine production wastewater, most acrylic acid is recovered by means of extraction and rectification and the like, various enriched organic materials with no application value are burned, the volume of the part to be burned is greatly reduced, the consumed fuel is less because the burning process is exothermic reaction, and the produced heat can be utilized. Low concentration waste water after being subjected to organic material extraction is treated by adopting a biochemical means. Compared with the prior art, the process of the present invention has the advantages that the energy consumption is significantly reduced and the material utilization ratio is improved.

## Claims

1. A method for treating methionine production waste water, comprising the following steps:
1) extracting the waste water with an extraction agent, and obtaining a first organic phase and a first water phase after the separation;
2) rectifying the first organic phase to obtain acrylic acid from the side line of the stripping section, obtain heavy components from the bottom of the column, and obtain the extraction agent containing light component(s) from the top of the column;
3) treating the extraction agent containing the light component(s) with a light component removal column, and separating to obtain the light component(s) and the extraction agent;
4) removing formaldehyde from the first water phase; and
5) biochemically treating the first water phase.

2. The method according to claim 1, wherein the light component(s) and/or the heave components obtained by separation are burned to recover heat.

3. The method according to claim 1, wherein the heavy components obtained by rectification are esterified and then separated.

4. The method according to claim 1, wherein the first water phase is stripped prior to Step 4) to recover the extraction agent.

5. The method according to claim 1, formaldehyde is saccharified by adding alkali and heating.

6. The method according to claim 5, wherein in the step of saccharifying, sodium hydroxide and calcium hydroxide are used as a mixed saccharifying agent by controlling pH value between 10-12 with sodium hydroxide and then adding sodium hydroxide, and the mass ratio of formaldehyde to calcium hydroxide is within a range of 5:1 to 1:2, and the saccharifiction temperature within a range of 60-120°C.

7. The method according to the claim 1, wherein in Step 4), formaldehyde is removed through a saccharification process which is performed in a steam stripping column.

8. The method according to claim 1, wherein, an alkali solution is used to remove the organic acid from the extraction agent containing light components prior to Step 3).

9. The method according to claim 8, wherein sodium hydroxide or calcium hydroxide aqueous solution is used to treat the extraction agent containing light components, and the second water phase obtained after separation is mixed with the first water phase.

10. The method according to any one of claims 1-7, wherein the extraction agent is selected from a group consisting of Methyl Isobutyl Ketone, Diisobutyl Ketone, ethyl acetate, isopropyl acetate, isobutyl acetate, methyl acrylate, ethyl acrylate, butyl acrylate, dimethylbenzene, toluene, heptane, hexahydrotoluene and isobutyl ether.

11. The method according to claim 10, wherein the extraction agent is Methyl Isobutyl Ketone.

12. The method according to claim 10, wherein during Step 2), an amount of water is added into the column reactor to enable water and Methyl Isobutyl Ketone to form an azeotrope, which is distilled out of the top of the column without containing acetic acid, and a small portion of the extraction agent entraining acetic acid is separated out from the upper side line of the column

## Patentansprüche

1. Verfahren zur Behandlung von Abwasser aus der Methioninproduktion, umfassend die folgenden Schritte:
1) Extrahieren des Abwassers mit einem Extraktionsmittel und Erhalten einer ersten organischen Phase und einer ersten Wasserphase nach der Trennung;
2) Rektifizieren der ersten organischen Phase, um aus der Seitenleitung der Abscheidungssektion Acrylsäure zu erhalten, schwere Komponenten aus dem Kolonnensumpf zu erhalten, und das Extraktionsmittel, das eine leichte Komponente/leichte Komponenten enthält, aus dem Kolonnenkopf zu erhalten;
3) Behandeln des Extraktionsmittels, das die leichte Komponente/leichten Komponenten enthält, mit einer Kolonne zur Entfernung leichter Komponenten, und Trennen, um die leichte Komponente/leichten Komponenten und das Extraktionsmittel zu erhalten;
4) Entfernen von Formaldehyd aus der ersten Wasserphase; und
5) biochemisches Behandeln der ersten Wasserphase.

2. Verfahren nach Anspruch 1, wobei die leichte Komponente/leichten Komponenten und/oder die schweren Komponenten, die durch die Trennung erhalten werden, verbrannt werden, um Wärme zurückzugewinnen.

3. Verfahren nach Anspruch 1, wobei die durch die Rektifikation erhaltenen schweren Komponenten verestert und dann abgetrennt werden.

4. Verfahren nach Anspruch 1, wobei die Wasserphase vor dem Schritt (4) gestrippt wird, um das Extraktionsmittel zurückzugewinnen.

5. Verfahren nach Anspruch 1, wobei Formaldehyd durch Zugeben von Alkali und Erwärmen verzuckert wird.

6. Verfahren nach Anspruch 5, wobei bei dem Schritt des Verzuckerns Natriumhydroxid und Calciumhydroxid als ein gemischtes Verzuckerungsmittel verwendet werden, indem der pH-Wert zwischen 10 - 12 mit Natriumhydroxid gesteuert wird und dann Natriumhydroxid zugegeben wird, und das Massenverhältnis von Formaldehyd zu Calciumhydroxid in einem Bereich von 5:1 bis 1:2 liegt, und die Verzuckerungstemperatur in einem Bereich von 60 - 120°C liegt.

7. Verfahren nach Anspruch 1, wobei in Schritt 4) Formaldehyd durch einen Verzuckerungsprozess entfernt wird, der in einer Dampfstrippkolonne durchgeführt wird.

8. Verfahren nach Anspruch 1, wobei eine Alkalilösung verwendet wird, um die organische Säure aus dem Extraktionsmittel, das leichte Komponenten enthält, vor Schritt 3) zu entfernen.

9. Verfahren nach Anspruch 8, wobei eine wässrige Lösung von Natriumhydroxid oder Calciumhydroxid verwendet wird, um das Extraktionsmittel, das leichte Komponenten enthält, zu behandeln, und die zweite Wasserphase, die nach der Abtrennung erhalten wird, mit der ersten Wasserphase gemischt wird.

10. Verfahren nach einem der Ansprüche 1 bis 7, wobei das Extraktionsmittel ausgewählt ist aus einer Gruppe, bestehend aus Methylisobutylketon, Diisobutylketon, Ethylacetat, Isopropylacetat, Isobutylacetat, Methylacrylat, Ethylacrylat, Butylacrylat, Dimethylbenzol, Toluol, Heptan, Hexahydrotoluol und Isobutylether.

11. Verfahren nach Anspruch 10, wobei des Extraktionsmittel Methylisobutylketon ist.

12. Verfahren nach Anspruch 10, wobei während Schritt 2) eine Menge an Wasser in den Kolonnenreaktor gegeben wird, um zu ermöglichen, dass Wasser und Methylisobutylketon ein Azeotrop bilden, welches am Kolonnenkopf destilliert wird, ohne dass es Essigsäure enthält, und ein kleiner Anteil des Extraktionsmittels, der Essigsäure mitführt, aus der oberen Seitenleitung der Kolonne abgetrennt wird.

## Revendications

1. Procédé de traitement des eaux usées de la production de méthionine, comprenant les étapes suivantes :
1) extraction des eaux usées avec un agent d'extraction, et obtention d'une première phase organique et d'une première phase aqueuse après la séparation ;
2) rectification de la première phase organique pour obtenir de l'acide acrylique de la ligne latérale de la section de décapage, obtention des composants lourds du fond de la colonne, et obtention de l'agent d'extraction contenant le(les) composant(s) léger(s) du sommet de la colonne ;
3) traitement de l'agent d'extraction contenant le(les) composant(s) léger(s) avec une colonne d'enlèvement de composant léger et séparation pour obtenir le(les) composant(s) léger(s) et l'agent d'extraction ;
4) enlèvement du formaldéhyde de la première phase aqueuse ; et
5) traitement biochimique de la première phase aqueuse.

2. Procédé selon la revendication 1, dans lequel le(s) composant(s) léger(s) et/ou les composants lourds obtenus par séparation sont brûlés pour récupérer la chaleur.

3. Procédé selon la revendication 1, dans lequel les composants lourds obtenus par rectification sont estérifiés puis séparés.

4. Procédé selon la revendication 1, dans lequel la première phase aqueuse est décapée avant l'étape 4) pour récupérer l'agent d'extraction.

5. Procédé selon la revendication 1, le formaldéhyde est saccharifié par addition d'agents alcalins et chauffage.

6. Procédé selon la revendication 5, dans lequel dans l'étape de saccharification, de l'hydroxyde de sodium et de l'hydroxyde de calcium sont utilisés comme agent de saccharification mixte en contrôlant la valeur de pH entre 10 et 12 avec l'hydroxyde de sodium puis en ajoutant de l'hydroxyde de sodium, et le rapport en masse du formaldéhyde à l'hydroxyde de calcium se situe dans une plage de 5 : 1 à 1 : 2, et la température de saccharification dans une plage de 60 à 120 °C.

7. Procédé selon la revendication 1, dans lequel dans l'étape 4), le formaldéhyde est enlevé par un processus de saccharification qui est effectué dans une colonne de décapage à la vapeur.

8. Procédé selon la revendication 1, dans lequel, une solution alcaline est utilisée pour enlever l'acide organique de l'agent d'extraction contenant les composants légers avant l'étape 3).

9. Procédé selon la revendication 8, dans lequel la solution aqueuse d'hydroxyde de sodium ou d'hydroxyde de calcium est utilisée pour traiter l'agent d'extraction contenant les composants légers, et la seconde phase aqueuse obtenue après la séparation est mélangée avec la première phase aqueuse.

10. Procédé selon l'une quelconque des revendications 1 à 7, dans lequel l'agent d'extraction est choisi dans un groupe constitué par la méthyl isobutyl cétone, la diisobutyl cétone, l'acétate d'éthyle, l'acétate d'isopropyle, l'acétate d'isobutyle, l'acrylate de méthyle, l'acrylate d'éthyle, l'acrylate de butyle, le diméthylbenzène, le toluène, l'heptane, l'hexahydrotoluène, et l'isobutyl éther.

11. Procédé selon la revendication 10, dans lequel l'agent d'extraction est la méthyl isobutyl cétone.

12. Procédé selon la revendication 10, dans lequel durant l'étape 2), une quantité d'eau est ajoutée dans le réacteur à colonne pour permettre à l'eau et à la méthyl isobutyl cétone de former un azéotrope, qui est distillé du sommet de la colonne sans contenir d'acide acétique, et une petite partie de l'agent d'extraction entraînant l'acide acétique est séparé de la ligne latérale supérieure de la colonne.
